# EUROPÄISCHE PATENTSCHRIFT

(11) **EP 2 004 260 B1**
(45) Veröffentlichungstag und Bekanntmachung des Hinweises auf die Patenterteilung: **20.03.2019**
(21) Anmeldenummer: 07720075.6
(22) Anmeldetag: 13.04.2007
(51) Int. Cl.: A61M 15/00

(54) **MEDIKAMENTENMAGAZIN, SOWIE MEHRDOSISPULVERINHALATOR**
MEDICAMENT LOADER AND MULTI-DOSE POWDER INHALER
CHARGEUR DE MÉDICAMENT ET INHALATEUR DE POUDRE MULTI-DOSE

(30) Priorität: 13.04.2006 EP 06405161
(43) Veröffentlichungstag der Anmeldung: 24.12.2008
(73) Patentinhaber: BOEHRINGER INGELHEIM INTERNATIONAL GMBH, 55216 Ingelheim am Rhein (DE)
(72) Erfinder: WACHTEL, Herbert, 55218 Ingelheim am Rhein (DE); GESER, Johannes, 55218 Ingelheim am Rhein (DE); METZGER, Burkhard P., 55218 Ingelheim am Rhein (DE); SPALLEK, Michael, 55218 Ingelheim am Rhein (DE); KRUEGER, Michael, 55218 Ingelheim am Rhein (DE); KUNZE, Hubert, 44227 Dortmund (DE); MOSER, Achim, 09130 Chemnitz (DE); MOCK, Elmar, 2013 Colombier (CH); LANCI, Antonino, 3006 Bern (CH); KLOPFENSTEIN, André, 2520 La Neuveville (CH)
(74) Vertreter: Frei Patent Attorneys
(86) Internationale Anmeldenummer: PCT/CH2007/000178
(87) Internationale Veröffentlichungsnummer: WO 2007/118340

(56) Entgegenhaltungen:
- WO-A-2004/067069
- DE-A1- 19 835 940
- US-A- 5 497 763
- US-A1- 2005 172 962
- US-B1- 6 929 004

## Beschreibung

Die Erfindung liegt auf dem Gebiet von Medikamentenmagazinen, in welchen in einer einzelnen Folienbahn Taschen zur Aufnahme eines Medikaments gebildet sind, gemäss dem Oberbegriff des unabhängigen Patentanspruchs. Ebenfalls Gegenstand der Erfindung ist ein Mehrdosispulverinhalator. Nicht beansprucht, aber beschrieben sind eine Vorrichtung und ein Verfahren zum Öffnen des Medikamentenmagazins.

Aus dem Dokument US 2005/0172962 ist ein Folienblister bekannt, in welchem ein einzelnes Folienband zu einzelnen Medikamententaschen gefaltet ist. Das Band weist einseitig entlang seiner Länge Öffnungen auf. In diese Öffnungen greift ein Zahnrad ein, welches den Blister blockiert, sobald eine Medikamententasche ein Piezoelement erreicht hat. Eine Aufwickelrolle auf der gegenüberliegenden Seite des Piezoelements wickelt das Folienband auf. Die Rolle zieht dabei solange an der Folie bis die entsprechende Medikamententasche geöffnet ist und das darin befindliche Medikament mit Hilfe des Piezoelements aus der Folie geschüttelt werden kann. Dieser Blister und Öffnungsmechanismus weist mehrere Nachteile auf. Zum einen muss das Blisterband inklusive Tasche über das Zahnrad rollen. Dazu müssen Öffnungen in den Taschen und des übrigens Bandes genau übereinstimmen um nicht vom Zahnrad 'gehoben' zu werden. Auch lässt dies ein zuverlässiges Einrasten des Rades im Blister nach dem Durchgang der Tasche fraglich erscheinen. Zudem müssen die einzelnen Taschen um mehr als eine Taschenlänge voneinander beabstandet sein, um nicht auf dem Zahnrad aufeinander zu liegen. Dies begrenzt jedoch eine gewünschte hohe Dichte an hintereinander angeordneten Taschen. Des Weiteren erfordert der beschriebene Öffnungsmechanismus viel Platz aufgrund der weit auseinander liegenden Räder.

In den Schriften DE 198 35 940 und WO 2004/067069 sind Medikamentenmagazine offenbart, welche beidseitige Transportöffnungen oder -vertiefungen in den Randbereichen des streifenförmigen Magazins aufweisen. Diese Magazine sind jedoch als Folienblister gestaltet, in welchen eine Deckfolie durchgedrückt oder abgepeelt wird. Die in US 2005/0172962 genannten Nachteile betreffend einen Einfolien-Faltblister werden in diesen Schriften entsprechend nicht behoben.

US 5 497 763 A betrifft die intrapulmonale Abgabe von Arzneistoffen und insbesondere eine Einmalverpackung, die einen oder mehrere Behälter enthält, der bzw. die in eine Kassette eingesetzt werden kann bzw. können, welche in eine Vorrichtung einbezogen werden kann, die zur gesteuerten Abgabe vernebelter, fließfähiger, flüssiger Formulierungen verwendet wird.

US 6 929 004 B1 beschreibt einen Träger mit einem Blatt, das einen ersten Abschnitt und einen zweiten Abschnitt aufweist, und mit einer Tasche zur Aufnahme eines Produkts in dem ersten Abschnitt. Eine Falte zwischen dem ersten und dem zweiten Abschnitt ist derart gestaltet, dass der zweite Abschnitt eine Abdeckung für die Tasche bildet. Eine Verbindung zwischen der Abdeckung und der Tasche ist vorgesehen. Dabei eignet sich der Träger für die Aufnahme von Medikamenten.

Es ist deshalb Aufgabe der Erfindung, den bekannten Einfolien-Faltblister zu verbessern und genannte Nachteile zu beheben, so dass vielseitigere Möglichkeiten in Bezug auf die Handhabung des Folienblisters möglich sind.

Die Aufgabe wird durch das Medikamentenmagazin gelöst, wie es in dem unabhängigen Anspruch definiert ist.

Bevorzugte Ausführungsformen der Erfindung sind in den Unteransprüchen beschrieben.

Das erfindungsgemässe Medikamentenmagazin weist eine Mehrzahl von Medikamentendosen auf, wobei das Magazin aus einer einzigen Folienbahn gebildet ist, in welcher Taschen zur Aufnahme der Medikamentendosen geformt sind. Die Folienbahn weist für den Transport der Bahn auf mindestens einer Seite Öffnungen auf für den Eingriff von Transportstacheln. Dabei weist die Folienbahn im Bereich der Taschen eine gewisse Breite auf, welche kleiner ist als die Breite der Folie in übrigen Bereichen, wobei die Öffnungen in diesem breiteren Bereich der Folienbahn angeordnet sind.

Dies bietet den Vorteil, dass der Folienblister seitlich geführt werden kann, im wesentlichen unabhängig von der Position, Lage und Masse der einzelnen Taschen. Die Taschen können Platz sparend, z. B. schuppenförmig, übereinander liegen, ohne den Transport des Bandes zu beeinflussen. Auch können Transport- und Öffnungsmittel unmittelbar aneinander anschliessen oder in einem einzigen Element kombiniert werden. Die Taschen können dabei direkt neben einem Transport- und/oder Öffnungsmittel, beispielsweise in einem Zwischenraum zwischen zwei parallel angeordneten Rädern, angeordnet werden. Ein Folienblister und eine Vorrichtung, in welche ein solcher Folienblister eingebracht wird, können damit sehr Platz sparend gestaltet werden.

Eine Folienbahn weist nach ihrer Verarbeitung zu einem Medikamentenmagazin mindestens eine Tasche auf, in welcher ein Medikament, beispielsweise ein Pulver, eingebracht ist. Vor bzw. hinter der mindestens einen Tasche, bei mehreren Taschen zwischen diesen, ist das Folienband breiter und weist die Öffnungen für einen Transport des Blisters, vorzugsweise auch für ein Öffnen des Blisters auf. Der die Öffnungen aufweisende breitere Bereich des Blisters bildet einen Rand entlang des Magazins, welcher vorzugsweise im wesentlichen so breit ist wie die Öffnungen. Da die Öffnungen jedoch lediglich zwischen den Taschen angeordnet sind, ist die Breite der Öffnungen unkritisch: Es wird keine Medikamentenkammer durch das Anbringen von Öffnungen beschädigt oder die Grösse einer solchen Kammer durch die Grösse der Öffnungen beeinträchtigt.

Vorzugsweise befinden sich in beiden Rändern der Folienbahn Öffnungen. Dies erlaubt ein sehr kontrolliertes, insbesondere symmetrisches, Transportieren und gegebenenfalls auch Öffnen des Magazins bzw. einer Tasche. Im Bereich zwischen den Rädern kann eine oder mehrere Taschen frei hängen oder liegen, unabhängig von der Führung des Medikamentenmagazins durch die Stacheln.

Die Anordnung und Gestaltung der Öffnungen kann zum Transportieren, Arretieren und Öffnen eines Blisters verwendet werden. Es ist aber auch möglich diese für weitere Funktionen, z. B. eine Indexierung und/oder Kontrolle zu verwenden. Über unterschiedliche Grössen, z. B. Längen, oder Abständen von Öffnungen kann beispielsweise zwischen einzelnen Taschen unterschieden werden. Es ist auch möglich darüber eine Anzeige von verbrauchten bzw. noch verwendbaren Medikamentendosen zu steuern.

Die Medikamententaschen können symmetrisch in der Folienbahn eingebracht sein. Es ist aber auch möglich eine Seite der Tasche mit einer Vertiefung zu versehen und die Abdeckung dann als im wesentlich ebene Fläche zu gestalten, oder mit der Folie vorerst eine Schlaufe zu formen und diese dann durch geeignete Versiegelungen vor bzw. nach dem Füllen zu verschliessen.

Eine nicht beanspruchte Vorrichtung zum Öffnen eines Medikamentenmagazins weist mindestens einen bewegbaren Stachel zum Eingriff in eine dafür vorgesehene Öffnung des Magazins auf. Zudem weist sie Mittel für einen Transportschub eines Magazins und Mittel für einen Öffnungsschub eines Magazins zum Öffnen einer in einer Folienbahn eingebrachten Tasche auf. Dabei ist der mindestens eine Stachel derart angeordnet, dass er in Bezug auf eine Transportrichtung seitlich und beabstandet zur Tasche in die Folienbahn eingreift. Durch die unterschiedlichen Folienbreiten im Bereich der Taschen und zwischen den Taschen, wird der Stachel, oder beispielsweise auch ein, mehrere Stacheln aufweisendes Zahnrad, parallel und seitlich zu den Taschen geführt. Durch diese bezüglich Transportrichtung parallele, seitlich beabstandete, gegebenenfalls direkt nebeneinander oder leicht hintereinander versetzt verlaufende Führung von Stachel und Tasche, ist die Tasche neben dem Stachel im wesentlichen frei bewegbar. Ein Transportstachel ist bevorzugt ein Zahn eines Zahnrades, wobei in einer nicht beanspruchten Ausführungsform mindestens zwei Stacheln parallel zueinander und um mindestens eine Medikamententaschenbreite beabstandet voneinander angeordnet sind, derart, dass eine Tasche zwischen den Stacheln bzw. einem die Stacheln aufweisenden Transport- und/oder Öffnungsmittel im wesentlichen frei bewegbar ist. Ein Transport- und ein Öffnungsschub sind in einer nicht beanspruchten Ausführungsform zwei individuelle, nacheinander ausgeführte Verfahrensschritte.

In der nicht beanspruchten Vorrichtung zum Öffnen eines Medikamentenmagazins sind Transport- und Öffnungsmittel vorzugsweise in einem einzigen Mittel kombiniert, welches Mittel bevorzugt als diskontinuierliches Stachelrad, z. B. ein Segmentrad, ausgebildet ist.

Beim Transportieren und Öffnen eines Medikamentenmagazins greift mindestens ein Stachel in eine dafür vorgesehene Öffnung des vorzugsweise eine Mehrzahl von Medikamententaschen aufweisenden Medikamentenmagazins ein und bringt durch eine Transportbewegung das Magazin in eine Öffnungsposition. Der Teil des Magazins, welches sich vor einer Medikamententasche im Bereich einer Öffnungsposition befindet, wird festgehalten und der Teil des Magazins, welches sich nach dieser Medikamententasche befindet wird mittels dem mindestes einen Stachel weitertransportiert. Dadurch wird die Medikamententasche aufgezogen, in dem die aneinander befestigten Folienbereiche voneinander gelöst werden, und das in der Medikamententasche befindliche Medikament wird, beispielsweise zur Inhalation, frei gegeben.

Eine Medikamententasche wird vorzugsweise durch mindestens zwei in das Medikamentenmagazin eingreifende und sich relativ voneinander wegbewegende Stacheln geöffnet. Sind die Stacheln Teil eines Segmentrades, so gehören die Stacheln zu unterschiedlichen Segmenten des Rades und eine Medikamententasche wird durch ein Auseinander bewegen bzw. Öffnen dieser Segmente geöffnet. Eine Medikamentenkammer ist dabei zwischen den zwei Segmenten des Segmentrades angeordnet.

In einer nicht beanspruchten Ausführungsform der Vorrichtung und des Verfahrens findet ein Öffnungs- und ein Transportschub in derselben Richtung statt. Dabei liegt eine geöffnete Medikamentenkammer bevorzugt auch in derselben Ebene, wie das Medikamentenmagazin während dem Transport in die Öffnungsposition, insbesondere jedoch in derselben Ebene wie das Magazin mit der zu öffnenden Tasche während dem Öffnen.

Das erfindungsgemässe Medikamentenmagazin wird typischerweise in einer Medikamentenausgabevorrichtung, vorzugsweise einem Inhalator, wie beispielsweise einem Mehrdosispulverinhalator, verwendet. Die Dosenanzahl liegt dabei vorzugsweise in einem Bereich von 1 bis 100 oder bis 200 Einzeldosen, bevorzugt in einem Bereich von 1-60, beispielsweise zwischen 7-180 oder 14-150, z. B. 30-120, 45-100, 30, 90, 60, 120. Für Inhalationsgeräte liegt eine Maximalanzahl von Einzeldosen aus Handlichkeits- und Therapie-Gründen vorzugsweise bei 60.

Ein erfindungsgemässer Mehrdosispulverinhalator weist somit ein erfindungsgemässes Medikamentenmagazin und optional eine Vorrichtung zum Öffnen eines solchen Magazins auf.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, HI-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroid, PDE4-Inhibitor, EGFR-Hemmer oder LTD4-Antagonisten,
- **W** stellt ein Anticholinergika dar, kombiniert mit einem Betamimetika, Corticosteroid, PDE4-Inhibitor, EGFR-Hemmer oder LTD4-Antagonisten,
- **W** stellt ein Corticosteroid dar, kombiniert mit einem PDE4-Inhibitor, EGFR-Hemmer oder LTD4-Antagonisten
- **W** stellt ein PDE4-Inhibitor dar, kombiniert mit einem EGFR-Hemmer oder LTD4-Antagonisten
- **W** stellt ein EGFR-Hemmer dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol,

Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2{([3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxyethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino }-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylaminol-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy} -propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino] -propyl} -phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 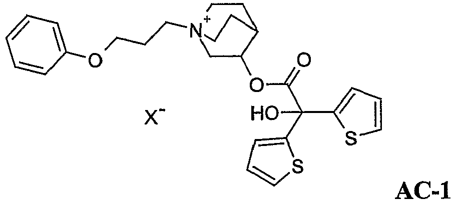 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 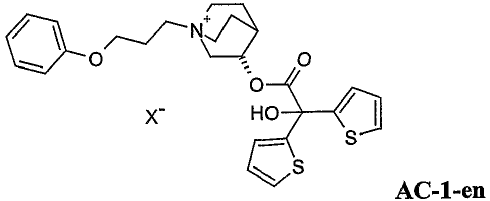 enthalten, worin X ⁻ die vorstehend genannten Bedeutungen aufweisen kann. Weiterhin bevorzugte Anticholinergika sind ausgewählt aus den Salzen der Formel **AC-2** 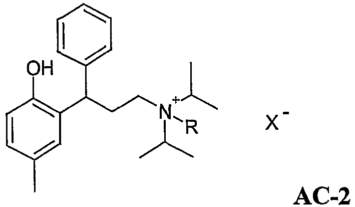 worin R entweder Methyl oder Ethyl bedeuten und worin X ⁻ die vorstehend genannte Bedeutungen aufweisen kann. In einer alternativen Ausführungsform kann die Verbindung der Formel **AC-2** auch in Form der freien Base **AC-2-base** vorliegen.

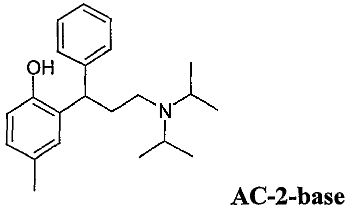

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3 '-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy)-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6- {trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino] -cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1 -yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4- {N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel, Arzneimittelformulierungen und - Mischungen mit den o.g. Wirkstoffen in Betracht, sowie deren Salze, Ester sowie die Kombination dieser Wirkstoffe, Salze und Ester.

Für die Herstellung von Medikamentenmagazinen werden bevorzugt pharmazeutisch zugelassene Materialien verwendet. Als Folien für den Einfolien-Faltblister können Mehrschichtfolien verwendet werden, welche auch zur Herstellung konventioneller Blister geeignet sind. Dies sind in der Regel Mehrschichtfolien, aufweisend eine Schicht PE, PP oder PVC und eine Aluminiumschicht. Je nach Anforderung ist die Folie entsprechend aufgebaut, beispielsweise stabiler, z.B. falls Vertiefungen darin geformt werden sollen. Die Folie ist zudem reissfest, beispielsweise durch den Einbau einer PET Schicht, so dass eine Medikamentenkammer durch ziehen am Folienband geöffnet werden kann, ohne zu reissen.

In einer bevorzugten Ausführungsform weist die Folie eine äussere, bezüglich Blister eine innenliegende, siegelbare Schicht auf. So können Randbereiche von Taschen durch Einbringen von Wärme verschweisst/verschmolzen werden.

Es ist auch möglich Siegellack, z.B. Heisssiegellack, zu verwenden. Dies erfordert einen zusätzlichen Arbeitsschritt zur Aufbringung des Lacks. Dafür sind dadurch mehr bzw. andere Materialien oder Materialkombinationen der Folie oder Folienschichten möglich.

Zum Siegeln wird Wärme an die entsprechenden zu verschweissenden/zu versiegelnden Stellen gebracht. Dies kann durch unterschiedliche Verfahren geschehen, beispielsweise mittels Heissstempel oder auch mittels Induktion, wobei eine Aluminiumschicht dabei als Induktionsschicht dienen kann und die Wärme an die umgebende Kunststoffschicht, welche als separate Lackschicht, als integrierte Lack-Folienschicht oder auch als Folienschicht gestaltet ist, abgibt.

Im Folgenden wird die Erfindung anhand von Beispielen und Figuren weiter erläutert, es zeigen:
- Fig. 1: einen Einfolien-Faltblister
- Fig. 2: eine nicht beanspruchte Abfolge eines Öffnungsvorganges eines Einfolien-Faltblisters
- Fig. 3: eine weitere Ausführungsform eines Einfolien-Faltblisters
- Fig. 4: einen nicht beanspruchten Transport- und Öffnungsmechanismus in der Form eines Segmentrades
- Fig. 5a-d: Verschiedene Öffnungspositionen eines Faltblisters

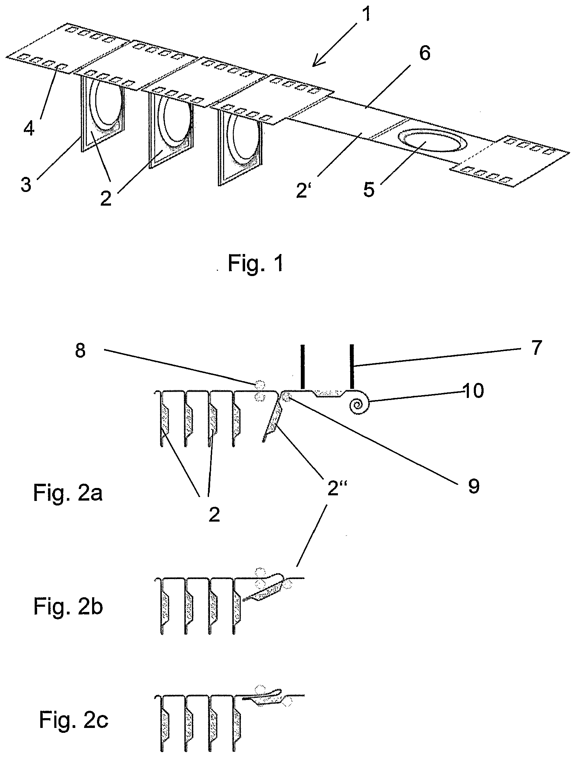

**Figur 1** zeigt einen Faltblister 1, welcher aus einem einzelnen Folienband hergestellt ist. Im Folienband sind in regelmässigen Abständen Taschen 2 geformt, welche vorzugsweise ein pulverförmiges Medikament beinhalten. Um ein Austreten von Medikament aus den Taschen zu verhindern, sind diese an ihren Rändern versiegelt (Siegelung 3). Eine geöffnete Medikamententasche 2' weist auf der einen Seite der Tasche eine napfförmige Vertiefung 5 im Folienband auf, während die andere Seite eine im wesentlichen ebene Abdeckung ist. Diese Art der Foliengestaltung erlaubt ein Füllen der Taschen, bei dem im wesentlichen jeweils nur die Abdeckung bewegt, d. h. aufgebracht und entfernt, werden muss und z. B. ein Pulver, auch beim oder nach einem Öffnen einer Tasche, ohne Gefahr zu laufen herauszufallen, in der Vertiefung liegt.

Die Taschen sind parallel zueinander und senkrecht zur Bandlängsachse angeordnet, wobei eine Transportrichtung des Blisters typischerweise auch entlang der Bandlängsachse verläuft. Dieses hintereinander Anordnen der Taschen, erlaubt eine sehr hohe Packungsdichte.

Der Bereich 6 des Folienbandes, welcher eine Tasche bildet, weist eine geringere Breite auf, als das übrige Folienband. In den breiteren, gegenüber den Taschen seitlich vorstehenden Bereichen des Folienbandes sind Öffnungen 4 eingebracht. Diese Öffnungen dienen zum Eingreifen von Transport- oder Öffnungsstacheln eines entsprechenden Transport- und/ oder Öffnungsmittels, z.B. eines Rades.

Die Öffnungen befinden sich nur zwischen den einzelnen Taschen. Die Öffnungen sind derart angeordnet, dass sie nach einer Taschenbildung eine im wesentlichen kontinuierliche Reihe von Öffnungen bilden. Es ist jedoch möglich, z. B. durch unterschiedlich grosse Abstände, durch unterschiedlich grosse Öffnungen, durch Aussparungen etc. die einzelnen Taschen voneinander zu unterscheiden (Indizierung). Beispielsweise können mehrere gleich grosse Öffnungen für einen Transport des Blisters vorgesehen sein, jedoch nur eine einzelne grössere Öffnung pro Medikamententasche. Ein beispielsweise für eine Arretierung vorgesehener breiter Stachel kann entsprechend nur in diese grössere Öffnung eingreifen und damit ein Arretieren auslösen.

Aufgrund der speziellen Gestaltung des Blisters ist es möglich eine einzige seitliche Öffnung pro Tasche vorzusehen und benachbarte Taschen bis auf den Abstand für diese seitliche Öffnung direkt aneinander anschliessend anzuordnen.

Dadurch, dass die Taschenbereiche schmaler sind, kann der Folienblister seitlich geführt werden. Die gesamte Handhabung des Folienblisters ist im wesentlichen unabhängig von der Position und Lage der einzelnen Taschen. Insbesondere ein oder mehrere Transportstacheln eines oder mehrerer Transporträder können an beliebiger Lage/zu beliebigem Zeitpunkt in den Blister seitlich eingreifen. Der oder die Stacheln oder Räder können in Transportrichtung gesehen parallel zum Medikamentenmagazin angeordnet werden. Die Stacheln können parallel neben den Tasche geführt werden. Für ein Eingreifen des oder der Stacheln, muss keine genaue Position zwischen Taschen abgepasst werden. Auch ein Passieren einer Tasche über ein Zahnrad fällt weg. Die Taschen können Platz sparend, schuppenförmig übereinanderliegen, ohne den Transport des Bandes zu beeinflussen. Von besonderem Vorteil ist diese Unabhängigkeit auch für eine Übergabe, beispielsweise von einem Transport- auf ein Öffnungsrad: Beide können im wesentlichen direkt aneinander anschliessen. Die Taschen befinden sich dabei neben einem Rad oder in einem Zwischenraum zwischen zwei parallel angeordneten Rädern, der in der Regel sowieso aufgrund des Durchmesser eines solchen Rades vorhanden ist. Transport- und Öffnungsmechanismen können so nahe beieinander angeordnet werden. Eine Gesamtbreite des Medikamentenmagazins inkl. Transport- und Öffnungsmittel, kann damit auf die Breite der Folie beschränkt werden, ohne auf eine hohe Taschendichte zu verzichten und ohne dabei auch weitere Nachteile, wie beispielsweise eine Mehrfachaufgreifen von Taschen durch Transportstachel in Kauf zu nehmen. Die Anordnung bietet auch Vorteile in Bezug auf eine Medikamentenausgabevorrichtung, in welche ein solcher Folienblister eingebracht wird. Diese kann im wesentlichen unabhängig von einer Taschentiefe bzw. einem Taschenabstand gestaltet sein, insbesondere wenn ein Öffnungsmechanismus durch die Anordnung und/oder Gestaltung der seitlichen Öffnungen ausgelöst wird.

In den **Figuren 2a****-c** ist ein Ablauf eines Öffnungsvorganges eines Faltblisters schematisch dargestellt. In der **Figur 2a** ist ein Faltblister mit mehreren mit einem Medikament gefüllten Taschen 2, mit einer vordersten, als nächste zu öffnenden Tasche 2" und mit einer geöffneten Tasche gezeigt. Die geöffnete Tasche befindet sich in einer Entnahmeposition, welche schematisch durch eine Inhalationskammer 7 angedeutet ist.

Der Blister wird zwischen zwei Transporträdern 8, einem oberen und einem unteren, geführt. Diese können mit einem Verriegelungsmechanismus ausgestattet sein, um den Blister an einer gewünschten Position, d.h. nach dem Durchgang der zu öffnenden Medikamententasche 2", festzuhalten. Es ist auch möglich nur ein unteres oder oberes Transportrad 8 vorzusehen, wobei vorzugsweise auf beiden Längsseiten des Blisterbandes je ein Transportrad angeordnet ist. Der Blister bzw. die zu öffnende Medikamententasche wird vorzugsweise genau an einer Stelle angehalten, derart dass sie nach einem Öffnen direkt in einer Entnahmeposition zu liegen kommt. Während die Transporträder, in Bezug auf eine Transportrichtung des Blisters, hinter einer Tasche angeordnet sind, ist, wiederum in Transportrichtung gesehen, vor der zu öffnenden Tasche ein Öffnungsrad 9 angebracht. Mit Hilfe des Öffnungsrades, welches vorzugsweise ebenfalls mittels Stacheln in die Öffnungen des Blisters eingreift und beidseitig des Blisters angeordnet ist, kann das Folienband vorderseitig weitergezogen und die Tasche 2" geöffnet werden. Dabei wird das Öffnungsrad, welches ein Zahnrad sein kann, um die zum Öffnen der Tasche benötigte Distanz gedreht. Aufgrund dessen, dass auch das bzw. die Öffnungsräder seitlich zur Medikamententasche angeordnet sind, kann eine mit Medikament gefüllte Vertiefung zwischen den zwei seitlich angeordneten Öffnungsrädern vorbeigezogen werden. Die Öffnungsräder dienen dabei auch als Führung.

Das Öffnungsrad kann im wesentlichen als Transportelement ausgestaltet sein und um eine entsprechende Distanz zusammen mit und parallel zu einem vorderen Teil des Folienbandes in Transportrichtung bewegt werden und dabei eine Tasche öffnen. Bevorzugt erfolgt ein Transport in die Öffnungsposition und ein Öffnen in individuellen Schritten nacheinander.

Die Zugkraft zum Öffnen der Taschen geht vorzugsweise vom Öffnungsrad aus, kann aber auch von einer Aufwickelrolle 10 ausgehen, auf welches das verbrauchte und geöffnete Folienband aufrollt wird. Eine Aufwickelrolle 10 wäre mit einem entsprechenden Schlupf versehen, um unterschiedliche Durchmesser ausgleichen zu können. Geht eine Zugkraft vom einem Öffnungsrad aus, kann das verbrauchte Folienband beispielsweise auch einfach in ein dafür vorgesehenes Volumen in einer Ausgabevorrichtung gelangen oder aus dieser austreten, wo das Band abgetrennt und entfernt werden kann.
Ein Öffnen kann im wesentlichen auch durch eine Aufwickelrolle ohne separates Öffnungsrad vorgenommen werden. Auch in diesem Fall weist das Aufwickelrad vorzugsweise einen Schlupf auf.

In den **Figuren 2b und 2c** ist die zu öffnende Tasche 2' in verschiedenen Transport- und Öffnungsstadien gezeigt. Dabei sind die Transport- und Öffnungsräder 8, 9 als Führungen für eine mit einer Vertiefung und Abdeckung versehene Tasche gestaltet. Aufgrund der unterstützenden Funktion des Öffnungsrades wird die Vertiefung in eine horizontale Lage gebracht und darin gehalten. Diese Massnahmen verhindern, dass während des Öffnens Pulver aus einer Tasche austreten kann, z.B. aufgrund einer Neigung des Folienblisters oder von allfälligen Kraftimpulsen, die während des Öffnens oder Transportierens auftreten könnten.

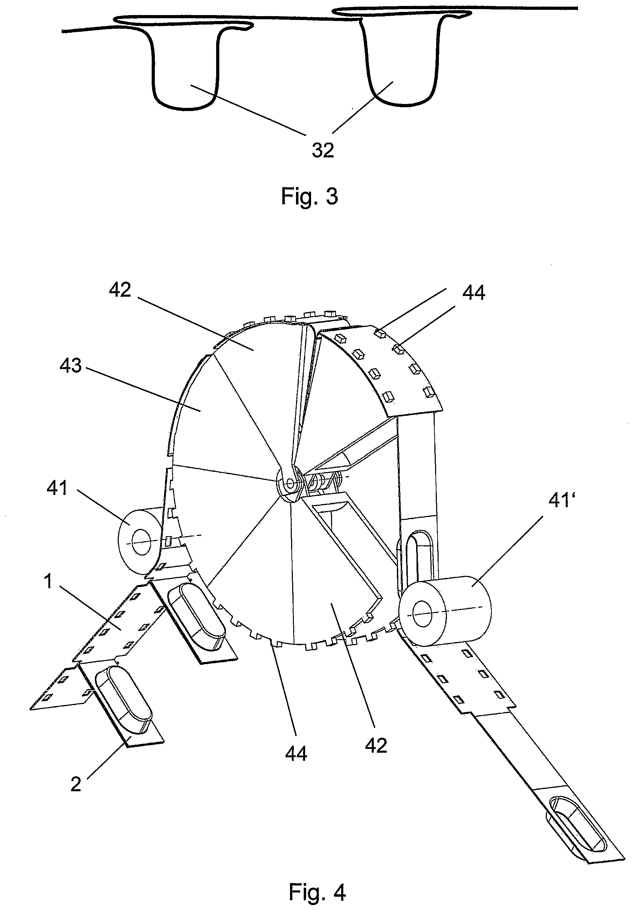

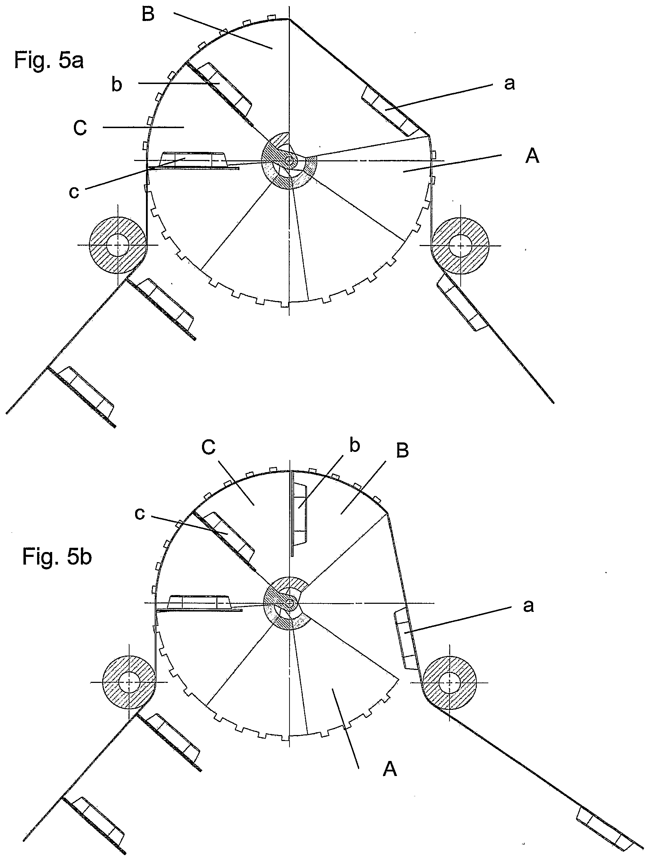

Ein Segmentrad kann das Transportieren und Öffnen des erfindungsgemässen Folienblisters, wie weiter unten in den Figuren 4 und 5a-d gezeigt, übernehmen.

Dabei kann sich zumindest der Abschnitt der Folienbahn mit der zu öffnenden Medikamententasche in derselben Ebene befinden, wie die geöffnete Medikamentenkammer, in welcher auch die Richtung des Transport- und Öffnungsschubes liegt.

Wird ein solches Medikamentenmagazin von zwei parallel angeordneten Zahnrädern geführt, so sind die im Magazin eingebrachten Taschen im Bereich zwischen den Zahnrädern im wesentlichen frei bewegbar. Eine Tiefe der Taschen wird gegebenenfalls durch eine die beiden Zahnräder führende Achse beschränkt. Sind tiefere Taschen oder kleine Zahnräder gewünscht, so können letztere beispielsweise von einer Aussenseite gelagert sein, so dass der gesamte Raum zwischen den Rädern für die Taschen frei ist, wie dies vorzugsweise auch bei nur einseitig geführten bzw. transportierten Einfolienfaltblistern der Fall ist.

In **Figur 3** ist eine weitere Gestaltung bzw. Herstellungsart eines Einfolien-Faltblisters gezeigt. Dabei wird mit einem Folienband eine Tasche 32 geformt. Die Tasche wird seitlich versiegelt (in der Figur nicht zu sehen), ein Medikament wird eingebracht und die Folie wird über die Einfüllöffnung der Tasche gelegt, wo sie ebenfalls angesiegelt wird und dabei die Tasche verschliesst. Schmalere Taschenbereiche und seitliche Öffnungen im Blister müssen nicht vorgängig im Folienband eingebracht werden, sondern können beispielsweise zeitgleich mit einer seitlichen Siegelung beziehungsweise mit einer oberen Siegelung der Taschen vorgenommen werden.
Allgemein ist ein Einfolien-Faltblister aus einem einzelnen Folienband hergestellt. Dabei ist eine Tasche oder eine Vertiefung mit zugehöriger Abdeckung aus ein und derselben Folie und aus aufeinander folgenden Folienabschnitten hergestellt.

In **Figur 4****,** sowie **Figur 5a****-d** ist ein Segmentrad und ein damit verbundener Öffnungsvorgang eines erfindungsgemässen Faltblisters dargestellt.

Der Folienblister 1 ist wiederum aus einem Folienband gebildet, in dem eine Vielzahl von Medikamententaschen 2 geformt ist. Das Folienband weist mehrere regelmässig hintereinander angeordnete Öffnungen in den seitlichen Randbereichen des Folienbandes zwischen den Taschen auf. Die Folienbereiche, welche die Taschen bilden, sind schmaler und weisen keine Öffnungen für Transportstacheln auf. Die Länge der Bereiche zwischen den Taschen korrespondiert mit der Länge eines Kreisbogensegments des Umfangs des Segmentrades 43, welches Kreisbogensegment durch eine Stirnseite eines einzelnen Segments 42 gebildet wird. Die Form und Anzahl der im Folienband eingebrachten Öffnungen korrespondieren mit Vorsprüngen, die als Transportstacheln dienen, hier jeweils vier Vorsprünge, die in zwei Reihen angeordnet sind. Die Vorsprünge befinden sich an zwei parallel zueinander angeordneten Segmentseiten und weisen radial nach aussen. Der Faltblister wird über die ineinandergreifenden Öffnungen und Transportstacheln über einen Teil des Segmentrades auf und wieder abgerollt.

Die einzelnen Segmente weisen einen U-förmigen Querschnitt auf, derart, dass eine Medikamententasche im Hohlraum zwischen den Segmentseiten zu liegen kommt. Die Segmentseiten, und damit die Transportstachelreihen oder einzelne Transportstacheln, sind um mindestens eine Taschenbreite voneinander beabstandet. Vorzugsweise sind sie nur wenig mehr, z.B. 0.1-2mm, als eine Taschenbreite voneinander beabstandet, um ein möglichst reibungsfreies Transportieren eines Blisters zu ermöglichen, um eine gute Führung zu bilden und um möglichst Platz sparend angeordnet zu sein. Das Segmentrad ist aus mehreren, einzeln bewegbaren Segmenten, hier sechs Segmenten, gebildet, wobei alle Segmente zusammengeführt, keinen vollständigen Kreis bilden. Der freie Radbereich, der zwischen zwei Segmenten gebildet wird, wird wenn von einem einzelnen Segment überstrichen, zum Öffnen einer Medikamententasche verwendet. Er ist entsprechend auf die Länge bzw. die doppelte Länge einer Medikamententasche eingestellt. Blisterband und Segmente sind zudem so aufeinander abgestimmt, dass eine Medikamententasche genau zwischen zwei Segmenten zu liegen kommt. Durch voneinander Trennen der beiden Segmente wird dabei der Folienabschnitt, welcher eine Medikamententasche bildet, auseinandergezogen und gibt die Medikamentenkammer und das darin befindliche Medikament frei.

Unmittelbar vor und nach dem Segmentrad sind zwei Halterollen 41, 41' angebracht, welche den Faltblister über und an dem Segmentrad in Position halten. Ein Öffnungsvorgang mittels des Segmentrades kann wie in den Figuren 5a-d gezeigt ablaufen. Der Faltblister wird über das Segmentrad geführt, wobei die Transportstacheln der einzelnen Segmente in die Öffnungen im Blister eingreifen. Durch eine Drehbewegung des Rades wird der Blister mit dem Rad transportiert. In **Figur 5a** ist der Blister in einer Entnahmeposition mit entleerter Medikamentenkammer a gezeigt. Die Entnahmeposition befindet sich in einem segmentfreien Bereich, wobei sämtliche Segmente aneinander anliegend weitertransportiert werden.

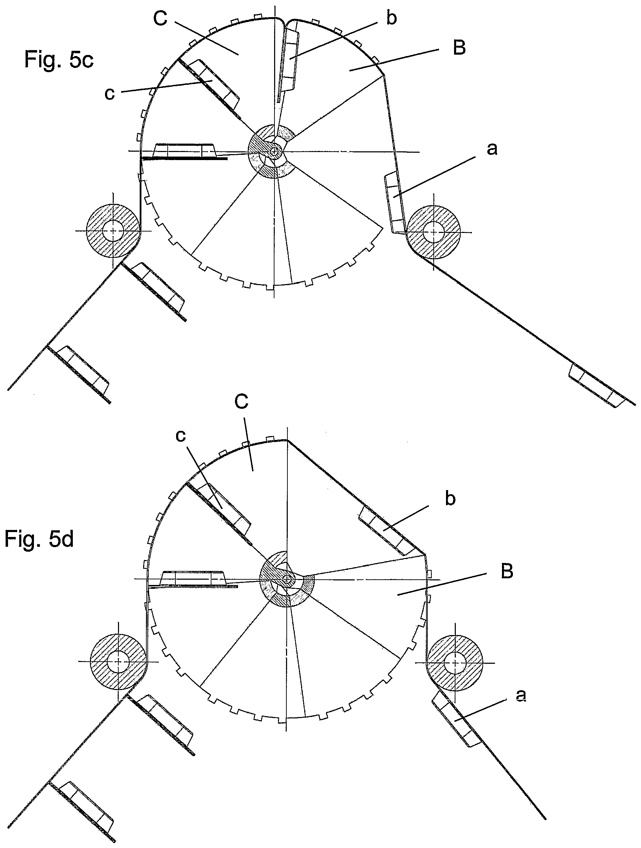

Die Drehbewegung des Segmentrades wird solange weitergeführt bis eine nächste Medikamententasche b in einer Öffnungsposition ist, wie in **Figur 5b** gezeigt. In diesem Punkt wird über einen Mechanismus, beispielsweise in der Nabe des Segmentrades, ein Arretiermechanismus ausgelöst, derart, dass das Blisterband vor der Öffnungsposition angehalten wird. Lediglich das nächste Segment B, auf das unmittelbar die als nächste zu öffnende Medikamententasche b folgt, wird in Transportrichtung weiterbewegt **(****Figur 5c**). Aufgrund der auf das Folienband ausgeübte Kraft, öffnet sich die Tasche b und kommt, wie in **Figur 5d** gezeigt, offen und zur Entnahme bereit, in der Entnahmeposition zu liegen. Die Vorsprünge des Segments B halten die Folie am Segment B, während die Vorsprünge am Segment C, die Folie am Segment C festhalten. Durch die Bewegung des Segments B weiter in Transportrichtung wird die Folie solange gezogen, bis sich die Siegelung zwischen den Folienbereichen der Medikamententaschen voneinander lösen und sich die Tasche öffnet.

Das nächste Segment B schliesst nun an die bis zu diesem Punkt stillstehenden übrigen Segmente des Rades an und das Verfahren gemäss Figur 5a-d kann mit der übernächsten Medikamentenkammer c und dem übernächsten Segment C wiederholt werden. Während dem Öffnungsprozess wird die verbrauchte Kammer a über die Halterolle 41' weggeführt, z. B. auf eine Aufwickelrolle aufgerollt oder aus einer Medikamentenvorrichtung ausgeführt, wo das Folienstück mit der verbrauchten Kammer entfernt werden kann.

Der Transport des Segmentrades, inklusive dem Öffnungsmechanismus einer Medikamententasche kann mit einem Öffnungsmechanismus einer Medikamentenausgabevorrichtung, beispielsweise einem Öffnen einen Mundstücks eines Mehrdosispulverinhalators kombiniert werden.

Das Segmentrad und die beiden Halterollen sind ortsfest und um ihre jeweilige Rotationsachse rotierbar in einer Medikamentenausgabevorrichtung, wie beispielsweise einem Inhalator, angebracht.

## Patentansprüche

1. Medikamentenmagazin mit einer Mehrzahl von Medikamentendosen, wobei das Magazin aus einer einzigen Folienbahn gebildet ist, in welcher Taschen (2, 32) zur Aufnahme eines Medikaments geformt sind, wobei die Folienbahn für den Transport der Bahn auf mindestens einer Seite Öffnungen (4) aufweist für den Eingriff von Transportstacheln, **dadurch gekennzeichnet, dass** die Folienbahn im Bereich der Taschen (2, 32) eine gewisse Breite aufweist, welche kleiner ist als die Breite der Folie in übrigen Bereichen, und dass die Öffnungen (4) in diesen breiteren Bereichen der Folienbahn angeordnet sind.

2. Medikamentenmagazin nach Anspruch 1, wobei sich die beiden Folienbreiten im wesentlichen um die Breite der Öffnungen (4) unterscheiden.

3. Medikamentenmagazin nach Anspruch 1 oder 2, wobei die Öffnungen (4) nur in Bereichen zwischen Taschen (2, 32) angeordnet sind.

4. Medikamentenmagazin gemäss einem der vorangehenden Ansprüche, wobei die Öffnungen (4) der Folienbahn für den Eingriff von Transportstacheln auch zum Öffnen der Taschen (2, 32) verwendbar sind.

5. Medikamentenmagazin gemäss einem der vorangehenden Ansprüche, wobei die Folienbahn auf beiden Längsseiten Öffnungen (4) aufweist.

6. Medikamentenmagazin gemäss einem der vorangehenden Ansprüche, wobei die Öffnungen (4) länglich sind und entsprechend ihrer Funktion unterschiedliche Längen aufweisen.

7. Medikamentenmagazin gemäss einem der vorhergehenden Ansprüche, wobei eine Tasche (2, 32) durch eine Vertiefung in der Folienbahn und eine im wesentlichen ebene Abdeckung gebildet ist.

8. Mehrdosispulverinhalator aufweisend ein Medikamentenmagazin gemäss einem der Ansprüche 1 bis 7.

9. Mehrdosispulverinhalator gemäss Anspruch 8, aufweisend eine Vorrichtung zum Öffnen des Medikamentenmagazins, wobei die Vorrichtung mindestens einen bewegbaren Stachel zum Eingriff in mindestens eine dafür vorgesehene Öffnung (4) des Magazins aufweist, und dass sie Mittel für einen Transportschub eines Magazins und Mittel für einen Öffnungsschub eines Magazins zum Öffnen einer in einer einzelnen Folienbahn eingebrachten Tasche (2, 32) aufweist, wobei der mindestens eine Stachel derart angeordnet ist, dass er in Bezug auf eine Transportrichtung parallel und seitlich zur Tasche (2, 32) in die Folienbahn eingreift und die Mittel für einen Transportschub eines Magazins und die Mittel für einen Öffnungsschub in einem diskontinuierlichen Stachelrad, z. B. einem Segmentrad (43), vereint sind.

10. Mehrdosispulverinhalator gemäss Anspruch 9, wobei ein als Segmentrad (43) ausgebildetes Stachelrad mehrere Segmente (42) aufweist, und ein maximaler Abstand zwischen zwei benachbarten Segmenten (42) im wesentlichen einer Bandlänge einer geöffneten Medikamententasche (2, 32) entspricht.

11. Mehrdosispulverinhalator gemäss einem der Ansprüche 8 bis 10 aufweisend 60 Medikamenteneinzeldosen.

12. Mehrdosispulverinhalator gemäss einem der Ansprüche 8 bis 11 zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer.

## Claims

1. Medicament magazine having a plurality of doses of medicament, the magazine being formed from a single foil strip, in which pouches (2, 32) for holding a medicament are formed, the foil strip comprising openings (4) on at least one side for transporting the strip, for the engagement of transporting pins, **characterised in that** the foil strip has a certain width in the region of the pouches which is less than the width of the foil in other regions, and **in that** the openings (4) are arranged in these broader regions of the foil strip.

2. Medicament magazine according to claim 1, the two foil widths essentially differing by the width of the openings (4).

3. Medicament magazine according to claim 1 or 2, wherein the openings (4) are arranged only in regions between pouches (2, 32).

4. Medicament magazine according to any one of the preceding claims, wherein the openings (4) in the foil strip for the engagement of transporting pins can also be used for opening the pouches (2, 32).

5. Medicament magazine according to any one of the preceding claims, wherein the foil strip has openings (4) on both longitudinal sides.

6. Medicament magazine according to any one of the preceding claims, wherein the openings (4) are elongated and have different lengths depending on their function.

7. Medicament magazine according to any one of the preceding claims, wherein a pouch (2, 32) is formed by a depression in the foil strip and a substantially planar cover.

8. Multi-dose powder inhaler comprising a medicament magazine according to any one of claims 1 to 7.

9. Multi-dose powder inhaler according to claim 8, comprising a device for opening the medicament magazine, wherein the device comprises at least one movable pin for engaging in at least one opening (4) provided for this purpose in the magazine, and comprising means for a transporting thrust of a magazine and means for an opening thrust of a magazine for opening a pouch (2, 32) formed in an individual foil strip, wherein the at least one pin is arranged such that it engages in the foil strip parallel to a transporting direction and laterally with respect to the pouch, the means for a transporting thrust of a magazine and means for an opening thrust being united in a discontinuous pin wheel, for example in a segmented wheel.

10. Multi-dose powder inhaler according to claim 9, wherein a pin wheel constructed as a segmented wheel (43) has a plurality of segments (42), and a maximum spacing between two adjacent segments (42) corresponds essentially to a band length of an opened medicament pouch.

11. Multi-dose powder inhaler according to any one of claims 8 to 10, comprising 60 single doses of medicament.

12. Multi-dose powder inhaler according to any one of claims 8 to 11, for administering a medicament which contains an active substance or a combination of active substances selected from among betamimetics, anticholinergics, steroids, antiallergics, ergot alkaloid derivatives, triptanes, CGRP antagonists, the phosphodiesterase-V inhibitors, phosphodiesterase-IV inhibitors, LTD4-antagonists, EGFR-kinase inhibitors.

## Revendications

1. Chargeur de médicament présentant plusieurs doses de médicament, le chargeur étant formé d'une seule nappe de film dans lequel des poches (2, 32) sont formées pour prendre un médicament, la nappe de film présentant sur au moins un côté des ouvertures (4) pour le transport de la nappe permettant l'engagement des ergots de transport,
**caractérisé en ce que**
la nappe de film présente au niveau des poches (2, 32) une largeur définie plus petite que la largeur du film dans les autres parties et
**en ce que** les ouvertures (4) sont disposées dans ces parties plus larges de la nappe de film.

2. Chargeur de médicament selon la revendication 1, dans lequel les deux largeurs du film diffèrent l'une de l'autre essentiellement de la largeur des ouvertures (4).

3. Chargeur de médicament selon les revendications 1 ou 2, dans lequel les ouvertures (4) ne sont disposées que dans les parties situées entre des poches (2, 32).

4. Chargeur de médicament selon l'une des revendications précédentes, dans lequel les ouvertures (4) de la nappe de film prévues pour l'engagement d'ergots de transport peuvent également être utilisées pour ouvrir les poches (2, 32).

5. Chargeur de médicament selon l'une des revendications précédentes, dans lequel la nappe de film présente des ouvertures (4) sur deux côtés longitudinaux.

6. Chargeur de médicament selon l'une des revendications précédentes, dans lequel les ouvertures (4) sont allongées et présentent des longueurs différentes selon leur fonction.

7. Chargeur de médicament selon l'une des revendications précédentes, dans lequel une poches (2, 32) est formée d'un creux ménagé dans la nappe de film et d'un recouvrement essentiellement plan.

8. Inhalateur de poudre multi-dose présentant un chargeur à médicament selon l'une des revendications 1 à 7.

9. Inhalateur de poudre multi-dose selon la revendication 8, présentant un dispositif d'ouverture du chargeur de médicament, le dispositif présentant au moins un ergot mobile qui s'engage dans une ou plusieurs ouvertures (4) du chargeur prévues dans ce but, et présentant des moyens d'avancement de transport du chargeur et des moyens d'avancement de l'ouverture du chargeur pour l'ouverture d'une poche (2, 32) formée dans une seule nappe de film, le ou les ergots étant disposés de manière à engager la nappe de film parallèlement à une direction de transport et latéralement par rapport à la poche (2, 32), les moyens d'avancement du chargeur et les moyens d'avancement de l'ouverture étant réunis dans une roue à ergots discontinue, par exemple une roue segmentée (43).

10. Inhalateur de poudre multi-dose selon la revendication 9, dans lequel une roue à ergots configurée comme roue segmentée (43) présente plusieurs segments (42), la distance maximale entre deux segments (42) voisins correspondant essentiellement à la longueur de bande d'une poche (2, 32) à médicament ouverte.

11. Inhalateur de poudre multi-dose selon l'une des revendications 8 à 10, présentant 60 doses individuelles de médicament.

12. Inhalateur de poudre multi-dose selon l'une des revendications 8 à 11, pour l'application d'un médicament qui contient une substance active ou une combinaison de substances actives sélectionnée dans l'ensemble constitué des bêtamimétiques, des anticholinergiques, des stéroïdes, des antiallergiques, des dérivés d'alcaloïdes d'ergot de seigle, des triptanes, des antagonistes du PRGC, des inhibiteurs de la phosphodiestérase-V, des inhibiteurs de la phosphodiestérase-IV, des antagonistes du LTD4 et des inhibiteurs de l'EGFR-kinase.
